# EUROPÄISCHE PATENTSCHRIFT

(11) **EP 0 518 190 B1**
(45) Veröffentlichungstag und Bekanntmachung des Hinweises auf die Patenterteilung: **02.08.2000**
(21) Anmeldenummer: 92109323.3
(22) Anmeldetag: 03.06.1992
(51) Int. Cl.: C07D 239/26, C09K 19/34, G02F 1/13

(54) **VERWENDUNG VON ALKENYLOXYPHENYLPYRIMIDINEN FÜR FERROELEKTRISCHE MISCHUNGEN**
USE OF ALKENYLOXYPHENYLPYRIMIDINES IN FERROELECTRIC COMPOSITIONS
UTILISATION D'ALKENYLOXYPHENYLPYRIMIDINES POUR MELANGES FERROELECTRIQUES

(30) Priorität: 14.06.1991 CH 177191; 14.06.1991 CH 177291; 16.10.1991 CH 303591
(43) Veröffentlichungstag der Anmeldung: 16.12.1992
(73) Patentinhaber: Rolic AG, 6301 Zug (CH)
(72) Erfinder: Fünfschilling, Jürg, CH-4054 Basle (CH); Kelly, Stephen, CH-4313 Möhlin (CH)
(74) Vertreter: Scott, Fiona Penelope Elaine

(56) Entgegenhaltungen:
- EP-A- 0 168 683
- WO-A-87/04426
- DE-A- 3 731 638

## Beschreibung

Die vorliegende Erfindung betrifft die Verwendung von Alkenyloxyphenylpyrimidin-Derivaten für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), SSF-Zellen (surface stabilized ferroelectric), DHF-Zellen (deformed helix ferroelectric) oder SBF-Zellen (short-pitch bistable ferroelectric).

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten und einen hohen Kontrast ergeben. Weiterhin sollten sie bei üblichen Betriebstemperaturen von etwa -30°C bis etwa +80°C, insbesondere von etwa -20°C bis etwa +60°C eine geeignete Mesophase besitzen, beispielsweise für die oben genannten Zellen eine breite smektische Mesophase.

Da Flüssigkristalle in der Regel als Mischungen mehrerer Komponenten zur Anwendung gelangen, ist es zudem wichtig, dass die Komponenten untereinander gut mischbar sind.

Gegenstand für die erfindungsgemässe Verwendung sind die Verbindungen der allgemeinen Formel 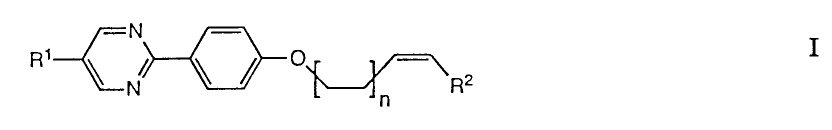
worin R¹ eine geradkettige Alkylgruppe mit 7 bis 10 Kohlenstoffatomen bedeutet; R² eine geradkettige Alkylgruppe mit 1 bis 8 Kohlenstoffatomen darstellt; und n entweder 1 oder 2 ist.

Flüssigkristalline Phenylpyrimidin Derivate sind in WO-A-87/04426, EP-A-0168683 und DE-A-3731638 beschrieben.

Es wurde überraschenderweise gefunden, dass die Verbindungen, für ferroelektrische Anwendungen, ausgesprochen günstige Mesophasen aufweisen. Durch die Einführung der cis-Doppelbindung in 3-, oder 5-Stellung der Alkoxyseitenkette der Verbindungen der Formel I wird oft eine bedeutende Absenkung des Schmelzpunktes bewirkt, was zu vergleichsweise breiten smektischen Mesophasen führt. Die smektischen Mesophasen der Verbindungen weisen eine überraschend niedrige Viskosität auf, was zu schnellen Schaltzeiten führt. Durch ihre Zumischung zu bekannten Grundkomponenten für ferroelektrische Mischungen wird das Auskristallisieren einzelner Komponenten signifikant unterdrückt, was zu einer vergleichsweise breiten Mesophase und einem ausgezeichneten Tieftemperaturverhalten führt. Ausserdem weisen Mischungen enthaltend die Verbindungen in SBF-Zellen ein ausgezeichnetes Multiplexverhalten auf.

Der Ausdruck "geradkettige Alkylgruppe mit 7 bis 10 Kohlenstoffatomen" bedeutet Heptyl, Octyl, Nonyl und Decyl. Der Ausdruck "geradkettige Alkylgruppe mit 1 bis 8 Kohlenstoffatomen" bedeutet Methyl, Aethyl, Propyl, Butyl, Pentyl, Hexyl, Heptyl und Octyl.

Bevorzugt werden Verbindungen der Formel I, worin R¹ Heptyl, Octyl oder Nonyl, R² Aethyl, Propyl, Butyl, Pentyl oder Hexyl und n 1 oder 2 bedeutet.

Ganz besonders bevorzugte Verbindungen der allgemeinen Formel I sind Verbindungen, worin R¹ Nonyl, n = 1 und R² Butyl, Pentyl oder Hexyl bedeutet, es sind dies die Verbindungen:
5-Nonyl-2-(4-[(3Z)-octenyloxy]phenyl)pyrimidin;
5-Nonyl-2-(4-[(3Z)-nonenyloxy]phenyl)pyrimidin;
5-Nonyl-2-(4-[(3Z)-decenyloxy]phenyl)pyrimidin;
   sowie Verbindungen der allgemeinen Formel I sind Verbindungen, worin R¹ Nonyl, n = 2 und R² Aethyl, Propyl oder Butyl; es sind dies die Verbindungen:
5-Nonyl-2-(4-[(5Z)-octenyloxy]phenyl)pyrimidin;
5-Nonyl-2-(4-[(5Z)-nonenyloxy]phenyl)pyrimidin;
5-Nonyl-2-(4-[(5Z)-decenyloxy]phenyl)pyrimidin.

Diese Verbindungen zeichnen sich durch einen besonders breiten smektischen Mesophasenbereich mit tiefem Schmelzpunkt aus.

Die Verbindungen können in an sich bekannter Weise z.B. aus 4-(5-Alkylpyrimidin-2-yl)phenol und dem in 3-, oder 5-Stellung ungesättigten Alkohol hergestellt werden. Die Reaktion erfolgt in Gegenwart von Azodicarbonsäure-diäthylester und Triphenylphosphin in Tetrahydrofuran oder einem anderen geeigneten Lösungsmittel, wie z.B. Hexan. Die als Ausgangsmaterialien verwendeten cis-Alkohole sind z.T. im Handel erhältlich oder können durch eine Wittig-Reaktion mit den kommerziell erhältlichen Aldehyden und einem Wittig-Reagenz (Alkyltriphenylphosphoniumchlorid) hergestellt werden. Vorzugsweise werden die cis-Alkohole jedoch durch katalytische Hydrierung der entsprechenden Alkinole in Gegenwart eines Lindlar-Katalysators hergestellt.

Die Verbindungen der Formel I können in Form von Gemischen untereinander und/oder mit anderen Flüssigkristallkomponenten verwendet werden. Die Verbindungen der Formel I werden jedoch vorzugsweise in Gemischen mit anderen Flüssigkristallkomponenten verwendet.

Die flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder auch andere flüssigkristalline Verbindungen sein. Es muss jedoch jeweils mindestens eine chirale Komponente im Gemisch enthalten sein. Sofern nicht bereits eine der verwendeten flüssigkristallinen Verbindungen selbst chiral ist, muss demnach ein chiraler Dotierstoff zugesetzt werden.

Derartige Flüssigkristallkomponenten sind vorzugsweise achirale Verbindungen der Formeln 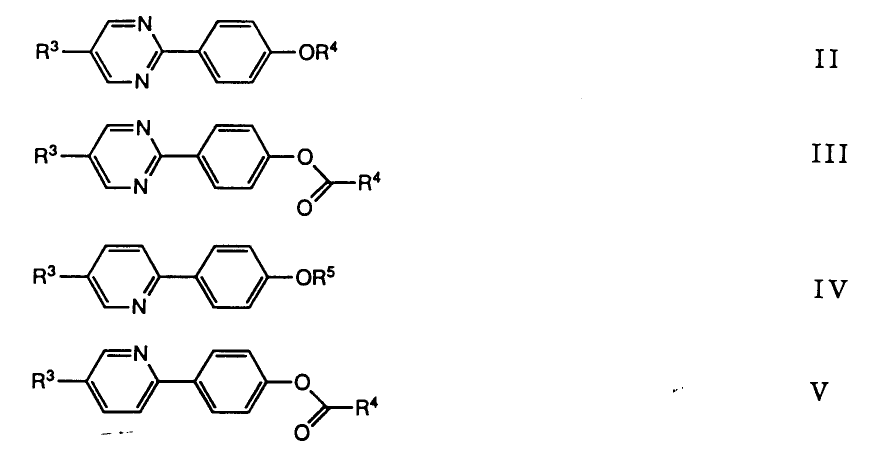 bzw. Dotierstoffe der allgemeinen Formeln 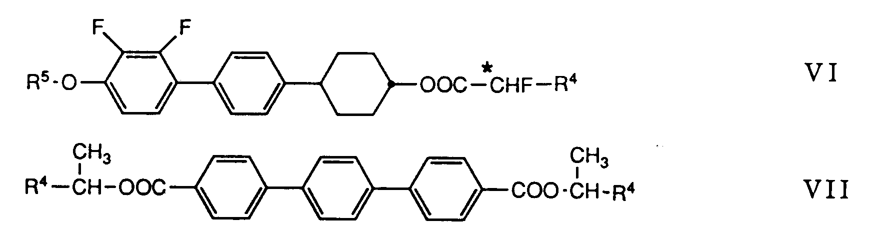
worin R³ Alkyl oder Alkoxy darstellt, R⁴ Alkyl bedeutet und R⁵ Alkyl oder Alkenyl darstellt.

Der Ausdruck"Alkyl" im Zusammenhang mit den Verbindungen der Formeln II bis VII umfasst unverzweigte oder verzweigte Alkylgruppen mit 1 bis 15 Kohlenstoffatomen, vorzugsweise unverzweigte Alkylgruppen mit 1-12 Kohlenstoffatomen wie Methyl, Aethyl, Propyl, Butyl, Pentyl, Hexyl, Heptyl, Octyl, Nonyl, Decyl, Undecyl, Dodecyl.

Der Ausdruck "Alkoxy" umfasst Aethergruppen in denen der Alkylrest wie vorhergehend definiert ist.

Der Ausdruck "Alkenyl" umfasst Alkenylgruppen mit 2 bis 15 Kohlenstoffatomen, wie 2E-Alkenyl-, 3Z-Alkenyl-, 4E-Alkenyl- und Alkenyle mit endständiger Doppelbindung. Die Ausdrücke "2E-Alkenyl", "3Z-Alkenyl" und 4E-Alkenyl" umfassen unverzweigte Alkenylgruppen mit 3 bis 15,4 bis 15 bzw. 5 bis 15 Kohlenstoffatomen, in welchen die Doppelbindung in 2, 3 bzw. 4 Stellung steht, wobei E und Z die Konfiguration der Doppelbindung bezeichnen. Solche Gruppen sind beispielsweise Allyl, 2E-Butenyl, 2E-Pentenyl, 2E-Hexenyl, 2E-Heptenyl, 2E-Octenyl, 2E-Nonenyl, 2E-Decenyl, 3-Butenyl, 3Z-Pentenyl, 3Z-Hexenyl, 3Z-Heptenyl, 3Z-Octenyl, 3Z-Nonenyl, 3Z-Decenyl, 4-Pentenyl, 4E-Hexenyl, 4E-Heptenyl, 4E-Octenyl, 4E-Nonenyl, 4E-Decenyl, 5-Hexenyl, 6-Heptenyl, 7-Octenyl, 8-Nonenyl, 9-Decenyl, 10-Undecenyl, 11-Dodecenyl und dergleichen.

Aufgrund der guten Löslichkeit der Verbindungen der Formel I und aufgrund ihrer guten Mischbarkeit untereinander kann der Anteil an Verbindungen der Formel I in den Gemischen relativ hoch sein und bis etwa 85 Gew.-% betragen. Bevorzugt wird im allgemeinen jedoch ein Anteil von etwa 1-50, insbesondere 5-30 Gew.-% an Verbindungen der Formel I.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische, S eine smektische und I die isotrope Phase.

### Beispiel 1

In einem Kolben wurden 0,5 g 5-Nonyl-2-(p-hydroxyphenyl)pyrimidin zusammen mit 0,22 g 3-(Z)-Nonen-1-ol, 0,29 g Azodicarbonsäure-diäthylester und 0,44 g Triphenylphosphin in 25 ml Tetrahydrofuran über Nacht bei Raumtemperatur gelöst. Die Lösung wurde eingeengt, der Rückstand mit 50 ml Hexan aufgeschlämmt und filtriert. Das Filtrat wurde eingeengt. Die chromatographische Reinigung des Rückstands an 25 g Kieselgel mit Hexan/Aethylacetat (9:1 Vol%) und anschliessende Umkristallisation aus 5 ml Aethylalkohol bei -25°C ergab 0,38 g reines 5-Nonyl-2-(p-(3Z)-nonenyloxyphenyl)pyrimidin mit einem Smp. (C-S_{C}) von 6°C, S_{C}-S_{A} 37°C und einem Klp. (S_{A}-I) von 51°C.

Auf analoge Weise wurden folgende Verbindungen hergestellt:
5-Heptyl-2-(4-[(3Z)-hexenyloxy]phenyl)pyrimidin, Smp. (C-S_{A}) 43°C, S_{A}-N 45°C, Klp. (N-I) 48°C;
5-Heptyl-2-(4-[(3Z)-octenyloxy]phenyl)pyrimidin, Smp. (C-S_{A}) 28°C, S_{A}-N 43°C, Klp. (N-I) 44°C;
5-Heptyl-2-(4-[(3Z)-nonenyloxy]phenyl)pyrimidin, Smp. (C-S_{C}) 25°C, S_{C}-S_{A} (6°C), Klp. (S_{A}-I) 40°C;
5-Heptyl-2-(4-[(3Z)-decenyloxy]phenyl)pyrimidin;
5-Heptyl-2-(4-[(3Z)-undecenyloxy]phenyl)pyrimidin;
5-Heptyl-2-(4-[(3Z)-dodecenyloxy]phenyl)pyrimidin;
5-Heptyl-2-(4-[(5Z)-octenyloxy]phenyl)pyrimidin, Smp. (C-S_{C}) 2°C, S_{C}-S_{A} 24°C, S_{A}-N 47°C, Klp. (N-I) 53°C;
5-Heptyl-2-(4-[(5Z)-nonenyloxy]phenyl)pyrimidin;
5-Heptyl-2-(4-[(5Z)-decenyloxy]phenyl)pyrimidin;
5-Heptyl-2-(4-](5Z)-undecenyloxy]phenyl)pyrimidin;
5-Heptyl-2-(4-[(5Z)-dodecenyloxy]phenyl)pyrimidin;
5-Octyl-2-(4-[(3Z)-hexenyloxy]phenyl)pyrimidin, Smp. (C-S_{C}) 42°C, S_{C}-S_{A} (32°C), Klp. (S_{A}-I) 47°C;
5-Octyl-2-(4-[(3Z)-octenyloxy]phenyl)pyrimidin, Smp. (C-S_{C})10°C, S_{C}-S_{A} 38°C, Klp. (S_{A}-I) 53°C;
5-Octyl-2-(4-[(3Z)-nonenyloxy]phenyl)pyrimidin, Smp. (C-S_{C}) 12°C, S_{C}-S_{A} 27°C, Klp. (S_{A}-I) 42°C;
5-Octyl-2-(4-[(3Z)-decenyloxy]phenyl)pyrimidin;
5-Octyl-2-(4-[(3Z)-undecenyloxy]phenyl)pyrimidin;
5-Octyl-2-(4-[(3Z)-dodecenyloxy]phenyl)pyrimidin;
5-Octyl-2-(4-[(5Z)-octenyloxy]phenyl)pyrimidin, Smp. (C-S_{C}) 25°C, S_{C}-S_{A} 34°C, Klp. (S_{A}-I) 45°C;
5-Octyl-2-(4-[(5Z)-nonenyloxy]phenyl)pyrimidin;
5-Octyl-2-(4-[(5Z)-decenyloxy]phenyl)pyrimidin;
5-Octyl-2-(4-[(5Z)-undecenyloxy]phenyl)pyrimidin;
5-Octyl-2-(4-[(5Z)-dodecenyloxy]phenyl)pyrimidin;
5-Nonyl-2-(4-[(3Z)-hexenyloxy]phenyl)pyrimidin, Smp. (C-S_{C}) 58°C, S_{C}-S_{A} (41°C), Klp. (S_{A}-I) (56°C);
5-Nonyl-2-(4-[(3Z)-octenyloxy]phenyl)pyrimidin, Smp. (C-S_{C}) 24°C, S_{C}-S_{A} 43°C, Klp. (S_{A}-I) 54°C;
5-Nonyl-2-(4-[(3Z)-decenyloxy]phenyl)pyrimidin;
5-Nonyl-2-(4-[(3Z)-undecenyloxy]phenyl)pyrimidin;
5-Nonyl-2-(4-[(3Z)-dodecenyloxy]phenyl)pyrimidin;
5-Nonyl-2-(4-[(5Z)-octenyloxy]phenyl)pyrimidin, Smp. (C-S_{C}) 24°C, S_{C}-S_{A} 45°C, Klp. (S_{A}-I) 61°C;
5-Nonyl-2-(4-[(5Z)-nonenyloxy]phenyl)pyrimidin;
5-Nonyl-2-(4-[(5Z)-decenyloxy]phenyl)pyrimidin;
5-Nonyl-2-(4-[(5Z)-undecenyloxy]phenyl)pyrimidin;
5-Nonyl-2-(4-[(5Z)-dodecenyloxy]phenyl)pyrimidin.

### Beispiel 2

Zur Untersuchung der Eigenschaften der Verbindungen der Formel I wurde eine Grundmischung hergestellt und jeweils 15% einer Verbindung der Formel I zugemischt. Die Phasenübergangstemperaturen dieser Mischungen wurden bestimmt, die Kristallisationstemperatur T_{c} wurde dabei aus Leitfähigkeitsdaten bestimmt. Die Schaltzeiten wurden bei 25°C (10 Vpp/µ, Zeit vom Start des Pulses bis zum Maximum des Stromes) gemessen. Die Messwerte sind in den Tabellen 1 und 2 zusammengefasst.

### Grundmischung

- 16 Gew.-%: p-[trans-4-{[(R)-2-Fluorhexanoyl]oxy}cyclohexyl]phenyl-2,3-difluor-4-(octyloxy)benzoesäureester;
- 24 Gew.-%: 2-[p-(Hexyloxy)phenyl]-5-nonylpyrimidin;
- 24 Gew.-%: 2-[p-(Nonyloxy)phenyl]-5-nonylpyrimidin;
- 12 Gew.-%: 2-[p-(Nonyloxy)phenyl]-5-heptylpyrimidin;
- 12 Gew.-%: 2-[p-(Heptyloxy)phenyl]-5-octylpyrimidin;
- 12 Gew.-%: 2-[p-(Decyloxy)phenyl]-5-octylpyrimidin.

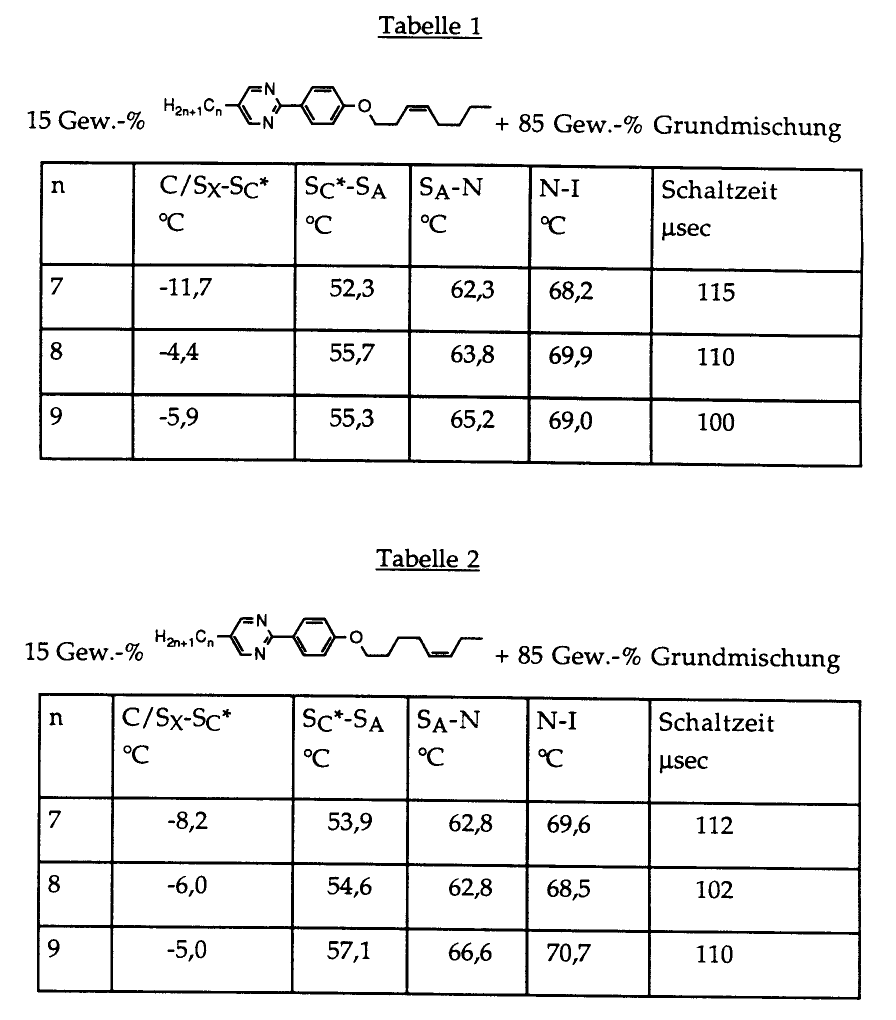

## Patentansprüche

1. Verwendung der Verbindungen der allgemeinen Formel 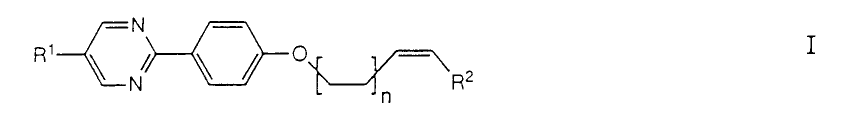
worin R¹ eine geradkettige Alkylgruppe mit 7 bis 10 Kohlenstoffatomen bedeutet, R² eine geradkettige Alkylgruppe mit 1 bis 8 Kohlenstoffatomen darstellt und n entweder 1 oder 2 ist in ferroelektrischen Mischungen.

2. Verwendung nach Anspruch 1, dadurch gekennzeichnet, dass R¹ Heptyl, Octyl oder Nonyl, insbesondere Nonyl, bedeutet.

3. Verwendung gemäss einem der Ansprüche 1 oder 2, dadurch gekennzeichnet, dass n = 1 und R² Butyl, Pentyl oder Hexyl bedeutet.

4. Verwendung gemäss einem der Ansprüche 1 oder 2, dadurch gekennzeichnet, dass n = 2 und R² Aethyl, Propyl oder Butyl bedeutet.

5. Verwendung gemäss Anspruch 1, dadurch gekennzeichnet, dass die Verbindung 5-Nonyl-2-(4- [(3Z)-octenyloxy]phenyl)pyrimidin, verwendet wird.

6. Verwendung gemäss Anspruch 1, dadurch gekennzeichnet, dass die Verbindung 5-Nonyl-2-(4-[(3Z)-nonenyloxy]phenyl)pyrimidin, verwendet wird.

7. Verwendung gemäss Anspruch 1, dadurch gekennzeichnet, dass die Verbindung 5-Nonyl-2-(4-[(3Z)-decenyloxy]phenyl)pyrimidin, verwendet wird.

8. Verwendung gemäss Anspruch 1, dadurch gekennzeichnet, dass die Verbindung 5-Nonyl-2-(4-[(5Z)-octenyloxy]phenyl)pyrimidin, verwendet wird.

9. Verwendung gemäss Anspruch 1, dadurch gekennzeichnet, dass die Verbindung 5-Nonyl-2-(4-[(5Z)-nonenyloxy]phenyl)pyrimidin, verwendet wird.

10. Verwendung gemäss Anspruch 1, dadurch gekennzeichnet, dass die Verbindung 5-Nonyl-2-(4-[(5Z)-decenyloxy]phenyl)pyrimidin, verwendet wird.

11. Verwendung von Flüssigkristallinen Gemischen gemäss Anspruch 1 mit mindestens 2 Komponenten, dadurch gekennzeichnet, dass mindestens eine Komponente eine Verbindung der in Anspruch 1 definierten Formel I ist.

## Claims

1. Use of the compounds of the general formula 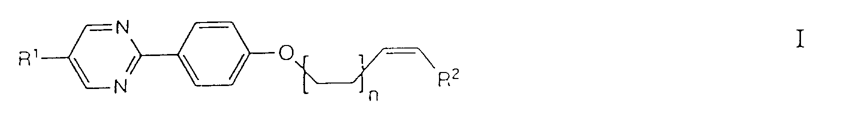
wherein R¹ represents a straight-chained alkyl group having from 7 to 10 carbon atoms; R² represents a straight-chained alkyl group having from 1 to 8 carbon atoms; and n is either 1 or 2, in ferroelectric mixtures.

2. Use according to claim 1, characterised in that R¹ represents heptyl, octyl or nonyl, especially nonyl.

3. Use according to either one of claims 1 and 2, characterised in that n = 1 and R² represents butyl, pentyl or hexyl.

4. Use according to either one of claims 1 and 2, characterised in that n = 2 and R² represents ethyl, propyl or butyl.

5. Use according to claim 1, characterised in that the compound 5-nonyl-2-(4-[(3Z)-octenyloxy]phenyl)pyrimidine is used.

6. Use according to claim 1, characterised in that the compound 5-nonyl-2-(4-[(3Z)-nonenyloxy]phenyl)pyrimidine is used.

7. Use according to claim 1, characterised in that the compound 5-nonyl-2-(4-[(3Z)-decenyloxy]phenyl)pyrimidine is used.

8. Use according to claim 1, characterised in that the compound 5-nonyl-2-(4-[(5Z)-octenyloxy]phenyl)pyrimidine is used.

9. Use according to claim 1, characterised in that the compound 5-nonyl-2-(4-[(5Z)-nonenyloxy]phenyl)pyrimidine is used.

10. Use according to claim 1, characterised in that the compound 5-nonyl-2-(4-[(5Z)-decenyloxy]phenyl)pyrimidine is used.

11. Use of liquid crystalline mixtures according to claim 1 having at least two components, characterised in that at least one component is a compound of the formula I defined in claim 1.

## Revendications

1. Utilisation des composés de formule générale 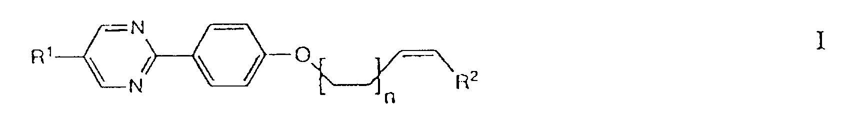
dans laquelle R¹ désigne un groupe alkyle à chaîne linéaire comportant 7 à 10 atomes de carbone, R² désigne un groupe alkyle à chaîne linéaire comportant 1 à 8 atomes de carbone et n représente 1 ou 2, dans des mélanges ferro-électriques.

2. Utilisation selon la revendication 1, caractérisée en ce que R¹ désigne un heptyle, un octyle ou un nonyle, en particulier un nonyle.

3. Utilisation selon l'une des revendications 1 ou 2, caractérisée en ce que n = 1 et R² désigne un butyle, un pentyle ou un hexyle.

4. Utilisation selon l'une des revendications 1 ou 2, caractérisée en ce que n = 2 et R² désigne un éthyle, un propyle ou un butyle.

5. Utilisation selon la revendication 1, caractérisée en ce que le composé 5-nonyl-2-(4-[(3Z)-octényloxy]phényl)pyrimidine est utilisé.

6. Utilisation selon la revendication 1, caractérisée en ce que le composé 5-nonyl-2-(4-[(3Z)-nonényloxy]phényl)pyrimidine est utilisé.

7. Utilisation selon la revendication 1, caractérisée en ce que le composé 5-nonyl-2-(4-[(3Z)-décényloxy]phényl)pyrimidine est utilisé.

8. Utilisation selon la revendication 1, caractérisée en ce que le composé 5-nonyl-2-(4-[(5Z)-octényloxy]phényl)pyrimidine est utilisé.

9. Utilisation selon la revendication 1, caractérisée en ce que le composé 5-nonyl-2-(4-[(5Z)-nonényloxy]phényl)pyrimidine est utilisé.

10. Utilisation selon la revendication 1, caractérisée en ce que le composé 5-nonyl-2-(4-[(5Z)-décényloxy]phényl)pyrimidine est utilisé.

11. Utilisation de mélanges pour cristaux liquides en conformité avec la revendication 1, avec au moins 2 composants, caractérisée en ce qu'au moins un composant est un composé répondant à la formule définie dans la revendication 1.
